# EUROPEAN PATENT SPECIFICATION

(11) **EP 2 473 224 B1**
(45) Date of publication and mention of the grant of the patent: **31.10.2018**
(21) Application number: 10814108.6
(22) Date of filing: 08.06.2010
(51) Int. Cl.: A61M 25/06

(54) **COAXIAL TRANSSEPTAL GUIDE-WIRE AND NEEDLE ASSEMBLY**
KOAXIALER TRANSSEPTALER FÜHRUNGSDRAHT UND NADELANORDNUNG DAFÜR
ENSEMBLE TRANSSEPTAL COAXIAL À FIL-GUIDE ET AIGUILLE

(30) Priority: 02.09.2009 US 239151 P
(43) Date of publication of application: 11.07.2012
(73) Proprietor: CircuLite, Inc., Teaneck, NJ 07666 (US)
(72) Inventor: FARNAN, Robert, C., River Vale NJ 07675 (US)
(74) Representative: Zimmermann & Partner Patentanwälte mbB
(86) International application number: PCT/US2010/037701
(87) International publication number: WO 2011/028310

(56) References cited:
- WO-A1-99/23958
- WO-A1-2009/055651
- WO-A2-2004/068947
- US-A- 5 947 940
- US-A1- 2002 038 129
- US-A1- 2002 038 129
- US-A1- 2002 143 291
- US-A1- 2003 125 709
- US-A1- 2004 092 867
- US-A1- 2005 070 881
- US-A1- 2005 101 984
- US-A1- 2005 101 984
- US-A1- 2007 016 165
- US-A1- 2007 185 530
- US-A1- 2007 232 898
- US-A1- 2007 293 724
- US-A1- 2008 097 398

## Description

### Cross-Reference to Related Application

### Technical Field

The present invention generally relates to devices for crossing a tissue and, more particularly, devices for percutaneously piercing through an internal tissue of the heart.

### Background

The circulatory system of the human body transports blood containing chemicals, such as metabolites and hormones, and cellular waste products to and from the cells. This organ system includes the heart, blood, and a vascular network. Veins are vessels that carry blood toward the heart while arteries carry blood away from the heart. A septum separates the left and right sides of the heart where each side includes an atrial chamber and a ventricular chamber. The atrial chambers receive blood from the veins and the ventricular chambers, which include larger muscular walls, pump blood from the heart. Movement of the blood is as follows: blood enters the right atrium from either the superior or inferior vena cava and moves into the right ventricle. From the right ventricle, blood is pumped to the lungs via pulmonary arteries to become oxygenated. Once the blood has been oxygenated, the blood returns to the heart by entering the left atrium, via the pulmonary veins, and flows into the left ventricle. Finally, the blood is pumped from the left ventricle into the aorta and the vascular network.

A number of surgical procedures are performed on the internal tissues of the heart, such as the implanting of a cardiac assist devices for treating congenital heart disease or valve procedures for repairing a prolapsing valve. Conventionally these procedures involved a thoracotomy, i.e., the opening of the thoracic cavity between successive ribs to expose the internal organs. More typical is cardiac surgery, generally known as open-heart surgery, where the sternum is cut and split to expose the internal organs. Once the thoracic cavity is accessed, the physician must enter the pleural space and puncture both the pericardium and the myocardial wall. There are great risks and an extensive recovery time associated with the invasive nature of the implantation surgery. As such, some patients with severe symptoms are not healthy enough for surgery to receive a circulatory assist system.

There have been some catheter-based procedures developed for accessing the chambers of the heart. Conventionally these procedures are performed from a vascular access site near the right femoral vein in order to accommodate the angle between the vena cava and the septum. Yet, there continues to be a need for improvements in crossing the septum and treating defects associated with the atrial septum (e.g., ASDs, PFOs).

US2005/0101984 discloses a device for performing a transseptal puncture procedure. In certain embodiments, the device includes a blunt outer needle, and a second inner needle disposed longitudinally through the lumen of the outer needle, wherein the inner needle is flexible, e.g., has a flexible portion and/or a bend or other non-traumatic conformation at its tip.

### Summary of the Invention

Claim 1 defines the invention and the dependent claims disclose the preferred embodiments. The invention provides a coaxial transseptal device comprising a piercing device comprising a shaft having a proximal end, a distal end, and a sharpened portion on the distal end, and a coaxial member having a proximal end, a distal end, and a lumen extending between the proximal and distal ends of the member, the lumen of the member configured to receive the piercing device and to move relative thereto; characterized in that the coaxial member is a guide-wire having a flexibility that increases distally, and in that a flexibility of the shaft of the piercing device increases distally, the shaft of the piercing device including a helical cut section on a distal end thereof for increasing the flexibility.

In another illustrative embodiment the guide-wire comprises a tube A coil surrounds at least the distal end of the tube and a hub is attached to the proximal end of the tube.

An exemplary method of piercing a tissue within the heart of a patient with the coaxial transseptal device is disclosed. The method includes introducing the distal end of the coaxial guide-wire into a superficial blood vessel. The distal end of the coaxial guide-wire is then directed through the superficial blood vessel and to the tissue within a first chamber of the heart. The needle portion of the piercing device is advanced beyond the distal end of the coaxial guide-wire, across the tissue, and into a second chamber of the heart. The distal end of the coaxial guide-wire is then advanced over the piercing device, across the tissue, and into the second chamber, which dilates the puncture in the tissue.

The guide-wire can include a removable adapter on the proximal end that can couple to a pressure monitor. The removable adapter and pressure monitor are configured to determine a pressure within a chamber of the heart.

### Brief Description of the Figures

FIGS. 1A and 1B are diagrammatic views of an exemplary method of accessing the septum of the human heart with a coaxial transseptal guide-wire and needle assembly, shown in cross-section.
FIG. 2A is a disassembled, side-elevational view of the coaxial transseptal guide-wire and needle assembly.
FIG. 2B is an assembled, side-elevational view of the distal end of the coaxial transseptal guide-wire and needle assembly.
FIG. 2C is an assembled, side-elevational view of the proximal end of the coaxial transseptal guide-wire and needle assembly with a pressure adaptor.
FIG. 3A is an assembled, side-elevational view of the distal end of the coaxial transseptal guide-wire and needle assembly, shown in partial cross-section.
FIG. 3B is an assembled, side-elevational view of an alternate embodiment of the distal end of the coaxial transseptal guide-wire and needle assembly, shown in partial cross-section.
FIGS. 4A-4B are side-elevational views of alternate embodiments of the distal end of a piercing device.
FIG. 4C is a cross-sectional view illustrating an alternate embodiment of the piercing device.
FIG. 5 is an assembled, side-elevational view of the coaxial transseptal guide-wire and needle assembly with a safety clip secured to the piercing device.
FIG. 6 is an assembled, side-elevational view of the coaxial transseptal guide-wire and needle assembly with the safety clip removed.
FIGS. 7A-7D are side-elevational views in partial cross section illustrating successive steps of one exemplary procedure for crossing the intra-atrial septum with the coaxial transseptal guide-wire and needle assembly.
FIG. 8 is a diagrammatic view of an alternate method of accessing the septum of the human heart with a coaxial transseptal guide-wire and needle assembly, shown in cross-section.

### Detailed Description

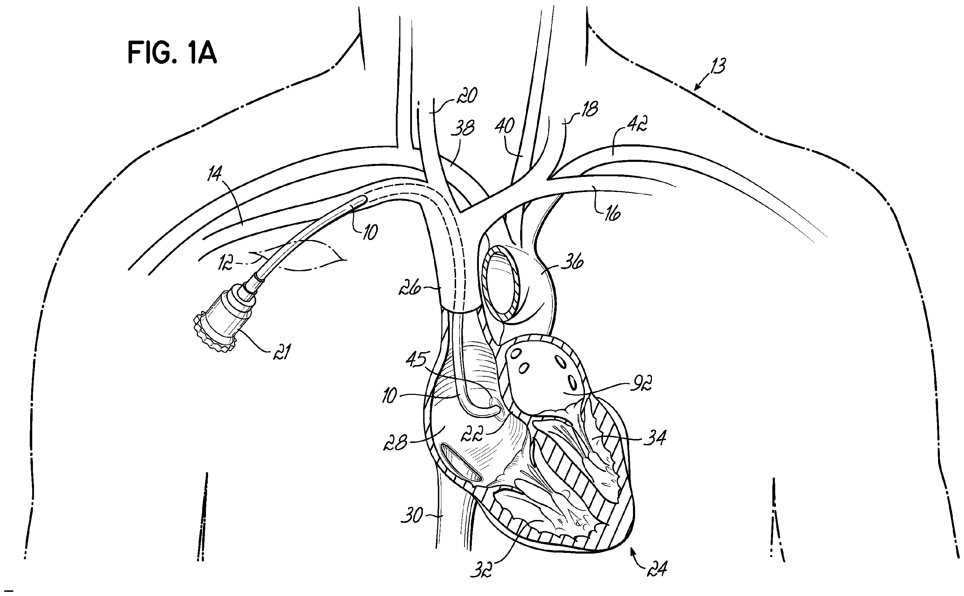

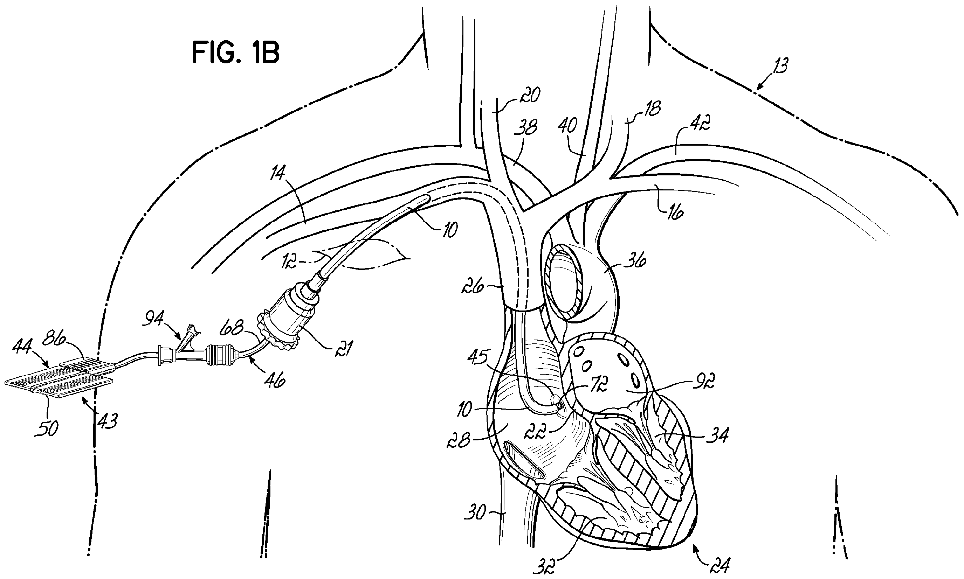

FIGS. 1A and 1B illustrate an exemplary method of transseptal crossing. Accordingly, the physician can direct a guide catheter 10 into a vascular access site 12 of the patient 13. The guide catheter 10 can be any steerable or preformed catheter that can be directed through the vascular system to aid in the delivery of subsequent surgical devices to the surgical site. The vascular access site can be near a suitable superficial blood vessel, such as the right subclavian vein 14; however, other superficial vessels could also be used, such as the left subclavian vein 16 or the left or right jugular veins 18, 20, or in some instances a superficial artery could also be used.

The guide catheter 10 can include a hub 21 having a hemostasis valve that prevents the loss of blood while maintaining access for passage of the subsequent devices.

The guide catheter 10 is directed to the intra-atrial septum 22 of the heart 24 via the superior vena cava 26 and the right atrium 28. For illustrative purposes additional anatomy is shown, including the inferior vena cava 30, the right ventricle 32, the left ventricle 34, the aortic arch 36, the brachiocephalic trunk 38, the left common carotid artery 40, and the left subclavian artery 42.

In FIG. 1B the distal end of the coaxial transseptal device 43 is directed into the guide catheter 10 at the vascular access site 12 and advanced through the lumen of the guide catheter 10 to the right atrium 28. Typically, the coaxial transseptal device 43 is advanced to an area of the right atrium 28 that is near the fossa ovalis 45.

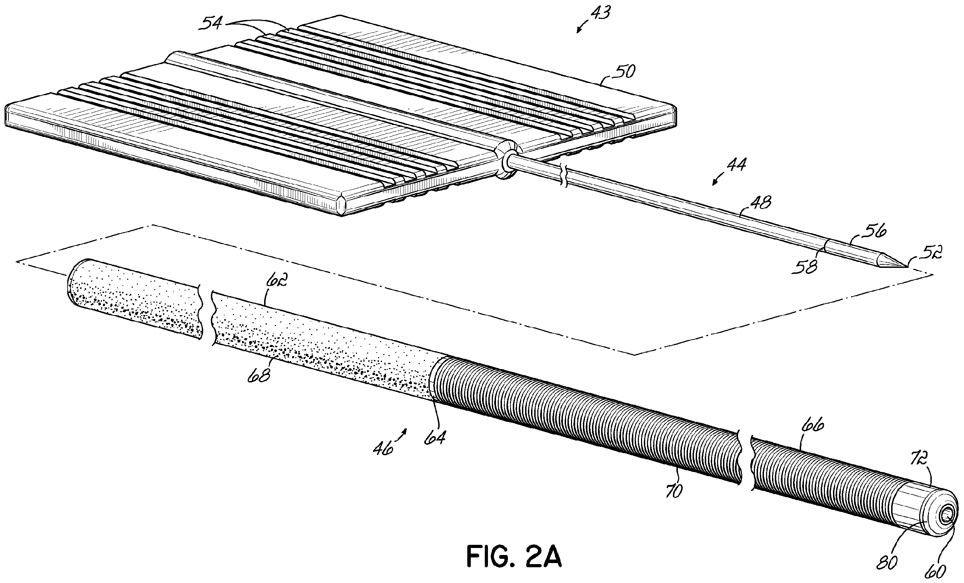

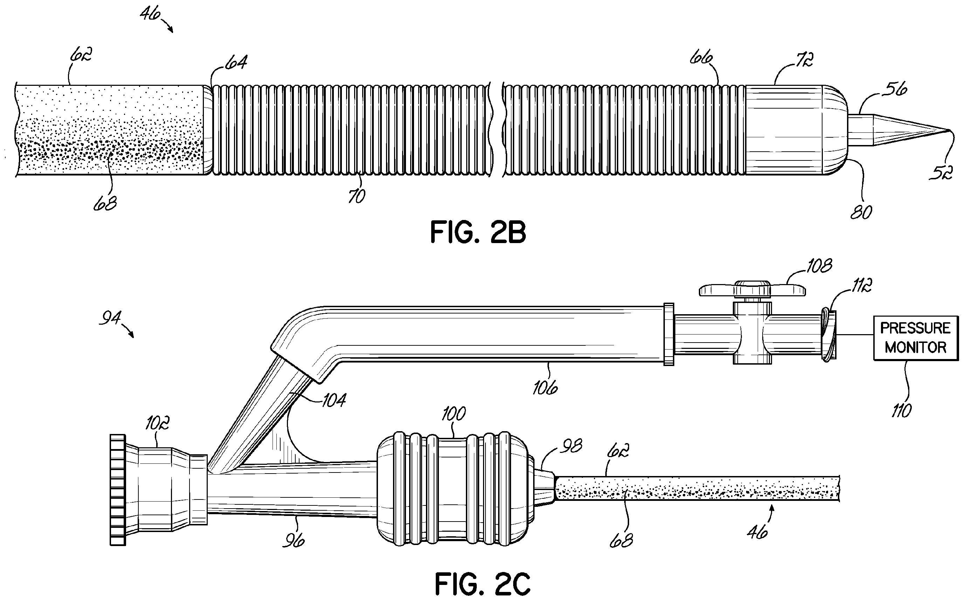

FIGS. 2A and 2B illustrate the details of the coaxial transseptal device 43, which includes a piercing device 44 and a coaxial guide-wire 46. The coaxial guide-wire 46 receives, and moves relative to, the piercing device 44.

As shown in FIG. 2A, the piercing device 44 includes a shaft 48 having distal and proximal ends, where the proximal end includes a hub 50 and the distal end includes a sharpened portion, i.e., a needle portion 52. The hub 50 can be secured to the flexible shaft 48 by either an insert injection molding process or by bonding a previously molded hub 50 to the shaft 48 using a biocompatible adhesive or epoxy. The hub 50 can include grooves 54 for providing additional grip between the physician's glove and the hub 50.

Referring still to FIG. 2A, the distal end of the shaft 48 can further include a radiopaque tip 56 constructed from any radiopaque material, such as platinum-iridium (Ptlr), stainless steel, tungsten (W), or tantalum (Ta). Radiopaque materials allow the physician to remotely visualize the structure, *in vivo*, by X-ray or real-time fluoroscopy. The distal end of the radiopaque tip 56 can be ground to an optimal shape for the needle portion 52, which facilitates the puncture and passage through a vascular tissue, such as the intra-atrial septum 22 (FIG. 1).

The shaft 48 can be constructed from metallic materials, such as MP35N, nickel titanium (NiTi), or stainless steel, or from a rigid polymer such as polyamide or polyaryletheretherketone (PEEK). To ensure visual contrast, the shaft material should not be as radiopaque as the material comprising the radiopaque tip 56. The joint 58 between the shaft 48 and the radiopaque tip 56 can be made by common welding techniques or by using a biocompatible adhesive or epoxy. The shaft 48 can also be coated with a lubricious polymer material to minimize friction between the flexible shaft 48 and the coaxial guide-wire 46. The shaft 48 is constructed with an outer diameter that is sized such that there is sufficient clearance between the outer diameter of the shaft 48 and the diameter of a lumen 60 extending through the coaxial guide-wire 46 to allow the shaft 48 to move relative to the coaxial guide-wire 46.

FIGS. 2A and 2B further illustrate the details of the coaxial guide-wire 46. The coaxial guide-wire 46 includes a proximal portion 62 that is separated by a transition joint 64 from a distal portion 66. The proximal portion 62 can include a proximal sleeve 68 constructed from a polymeric thermoset material, such as polytetrafluoroethylene (PTFE) or polyimide, or a thermoplastic polymer, such as fluorinated ethylene propylene (FEP), polyurethane, or polyamide. Generally, the material should have a low coefficient of friction or be a material that will accept a lubricious polymer material. While the thickness of the proximal sleeve 68 can vary and depend on a desired final outer diameter of the coaxial guide-wire 46, the wall thickness of the proximal sleeve 68 can vary from about 0.0127 mm (0.0005 inches) to about 3.81 mm (0.15 inches). Typical outer diameters for the guide-wire can range from about 0.3 mm (0.012 inches) to about 1.0 mm (0.038 inches).

The coaxial guide-wire 46 can vary in length to accommodate various surgical procedures, but generally range from about 50 cm to about 400 cm.

The distal portion 66 of the coaxial guide-wire 46 can include a coil 70 extending between a distal tip 72 and the proximal sleeve 68. The coil 70 can be constructed from a metallic material, such as stainless steel or Ptlr, and is typically round in cross-section, though rectangular or flat wire cross-sections are possible. The round cross-section coils can range in diameter from about 0.0254 mm (0.001 inches) to about 0.254 mm (0.010 inches); flat wire coils can have a thickness-to-width ratio ranging from about 1:2 to about 1:4 with thickness ranging from about 0.0127 mm (0.0005 inches) to about 0.127 mm (0.005 inches). The coil 70 can be coated with a lubricious material, such as PTFE. In some embodiments, though not shown here, the coil 70 can extend the full length of the coaxial guide-wire. The proximal sleeve 68 and coil 70 have similar outer diameters to ensure a smooth transition between components and are joined at the transition joint 64 by common welding techniques or by using a biocompatible adhesive or epoxy.

The distal tip 72 can be constructed from a dense metal to enhance its radiopacity and has an outer diameter that is substantially similar to the outer diameter of the coil 70 to ensure a smooth transition between the components.

FIG. 2C illustrates a removable adapter 94 that can be attached to the proximal end of the coaxial guide-wire 46. The removable adapter 94 includes a body 96 and a distal collet 98 with an adjustment mechanism 100 for tightening the collet 98 against the coaxial guide-wire 46. The collet 98 can include an internal ring (not shown) for creating a fluid tight seal against the coaxial guide-wire 46. The proximal end of the body 96 can include a hemostasis valve hub 102 to prevent the loss of blood while maintaining a fluidic access to the lumen 60 (FIG. 2A) of the coaxial guide-wire 46. The body 96 further includes a side port 104 with tubing 106 and a stopcock 108, which can then be attached to a pressure monitor 110. The pressure monitor 110 can allow the physician to monitor the pressure within the left atrium 92 (FIG. 1A) during the surgical procedure, particularly when puncturing the intra-atrial septum 22 (FIG. 1A). By monitoring the pressure within the left atrium 92 (FIG. 1A) the physician can ensure that the transseptal crossing has occurred in the appropriate location. A luer fitting 112 can be used for attaching the tubing 106 to the pressure monitor 110 or other device within the operating room.

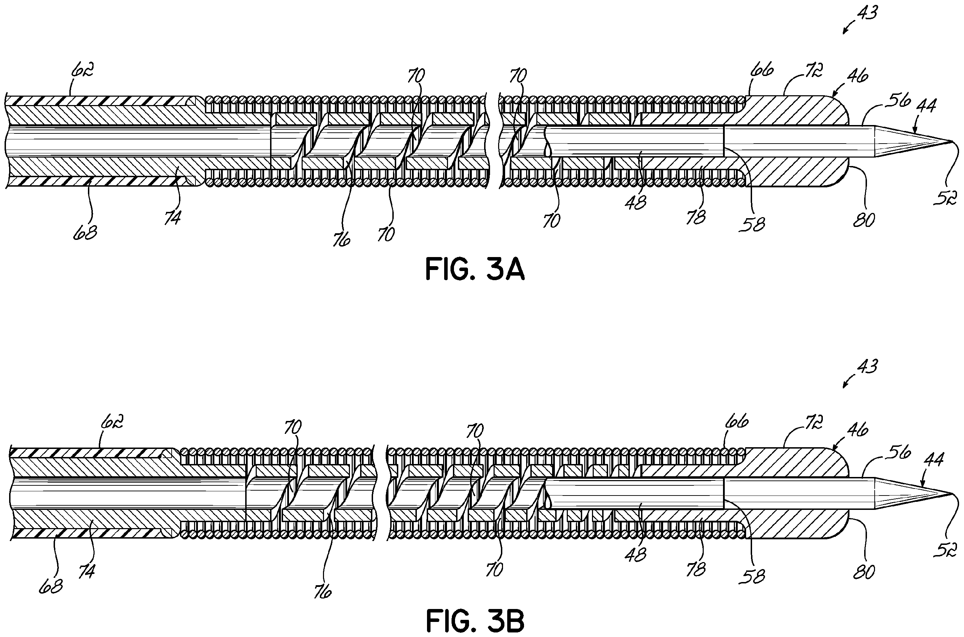

FIGS. 3A illustrates the cross-sectional features of the distal ends of the coaxial guide-wire 46 and the piercing device 44. The coaxial guide-wire 46 includes a tube 74 extending proximally from the distal tip 72. Construction of the tube 74 can include metallic materials, such as MP35N, NiTi, or stainless steel. The tube 74 is formed by a wire drawing process and electro-polished to remove sharp edges. While the wall thickness of the tube 74 can vary, typical thicknesses can range from about 0.0254 mm (0.001 inches) to about 0.254 mm (0.010 inches).

The distal portion of the tube 74 can be processed by laser into a spiral cut section 76 to provide a flexibility that increases distally. That is, the distal portions of the spiral cut section 76 are more flexible than the proximal portions of the spiral cut section 76. As shown in FIG. 3A, the spiral cut section 76 can have a uniform pitch at the distal end of the tube 74. Alternatively, as shown in FIG. 3B, the spiral cut section 76 can have a variable pitch such that the flexibility of the spiral cut section is further increased distally, i.e., the proximal section of the spiral cut section 76 has a larger pitch than the more flexible, distal section. The spiral cut section 76 allows the distal end of the coaxial guide-wire 46 to be flexible enough to pass through the vascular system while still limiting the radius of curvature. The spiral cut section 76 should be constructed with a direction that opposes the direction of the pitch of the coil 70 to prevent the coil 70 from penetrating into the spiral cut section 76 as the coaxial guide-wire 46 is passed through a bend in the vascular system.

The distal tip 72 includes a tip shoulder 78 and a radial tip 80. The tip shoulder 78 provides a surface for adjoining the distal tip 72, the coil 70, and the tube 74 by common welding techniques or by using a biocompatible adhesive or epoxy. The radial tip 80 minimizes unintentional trauma to vascular tissue as the coaxial guide-wire 46 is advanced through the vascular network. While a rounded shaped radial tip 80 is shown, it is possible for the radial tip 80 to alternatively include a bullet shape, a bevel, or an elliptical shape.

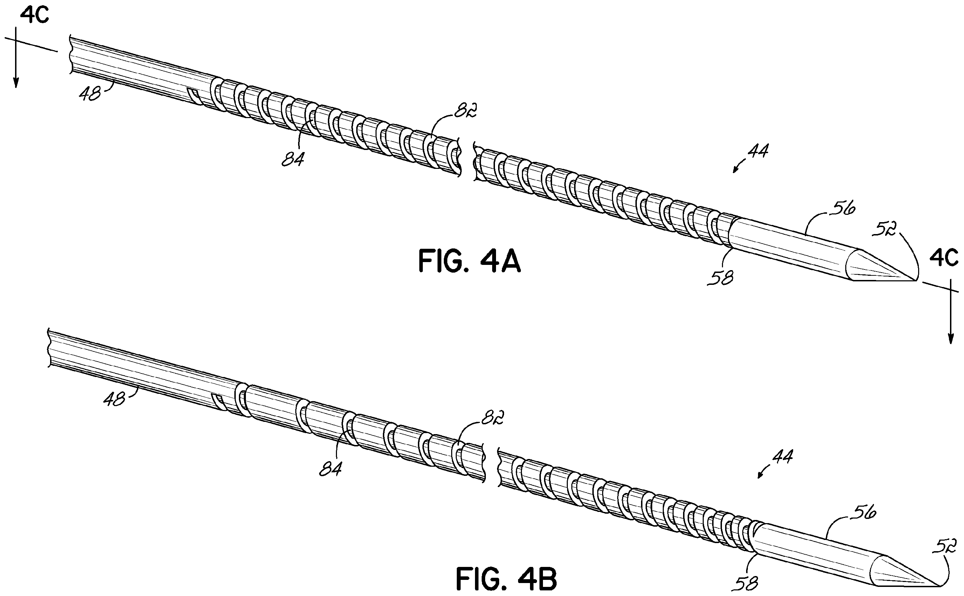

Turning now to FIG. 4A, one embodiment of the piercing device 44 is illustrated. As shown, the flexible shaft 48 can include a distally-positioned spiral cut section 82, which can be made by laser machining. The spiral cut section 82 is typically helical and can penetrate into the material of the shaft 48 by no more than ½ of the original outer diameter of the shaft 48. This would allow at least ½ of the original outer diameter to remain as a core 84. The core 84 provides structural stability to aid in advancing the piercing device 44 through the coaxial guide-wire 46. The spiral cut section 82 provides flexibility to the distal end of the piercing device 44 as it advances through the coaxial guide-wire 46 within the vascular network.

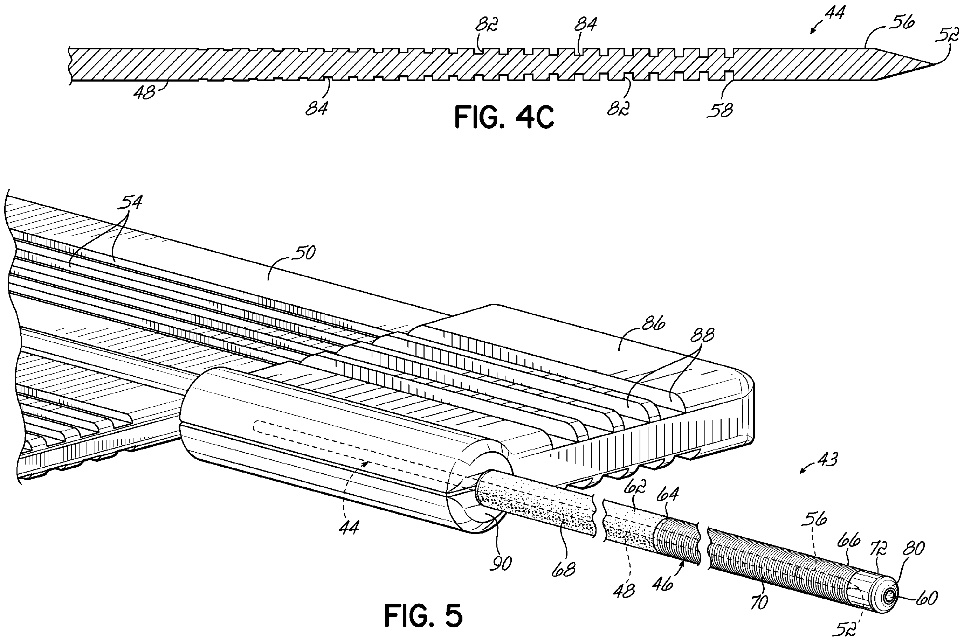

FIGS. 4B and 4C illustrate alternate embodiments of the piercing device 44. In FIG. 4B, the spiral cut section 82 is cut with a variable pitch, as compared to the constant pitch of FIG. 4A. Accordingly, the variable pitch is such that the proximal portion of the spiral cut section 82 of the piercing device 44 has a greater pitch and is less flexible than the distal portion of the spiral cut section 82. FIG. 4C illustrates an embodiment where the spiral cut section 82 is cut with a variable depth such that the distal portion of the spiral cut section 82 is cut deeper and is therefore more flexible than the proximal portion. At the most distal portion of the spiral cut section 82, the helical cut should not penetrate into the material of the shaft 48 by more than ½ of the original outer diameter.

FIG. 5 illustrates the assembled coaxial transseptal device 43. The piercing device 44 is back-loaded into the coaxial guide-wire 46 until the hub 50 is positioned near the proximal end of the coaxial guide-wire 46. To ensure that the needle portion 52 remains sheathed within the coaxial guide-wire 46, and to prevent inadvertent and premature puncture of the vascular tissue, a safety clip 86 can be positioned on the shaft 48 between the hub 50 and the proximal end of the coaxial guide-wire 46. The safety clip 86 can be machined or molded from a thermoplastic material, a polymer, or metal and can include grooves 88 to improve the grip between the physician's glove and the safety clip 86. In the illustrative embodiment, the safety clip 86 is constructed with an attachment portion 90 that snaps onto the flexible shaft 48, though additional safety locks and features could also be used. The attachment portion 90 creates a frictional fit against the shaft 48 and prevents the needle portion 52 from prematurely advancing beyond the distal end of the coaxial guide-wire 46. The length of the attachment portion 90 that contacts the shaft 48 will determine the penetration depth of the piercing device 44 into the left atrium 92 (FIG. 1A), in a manner that is described in greater detail below.

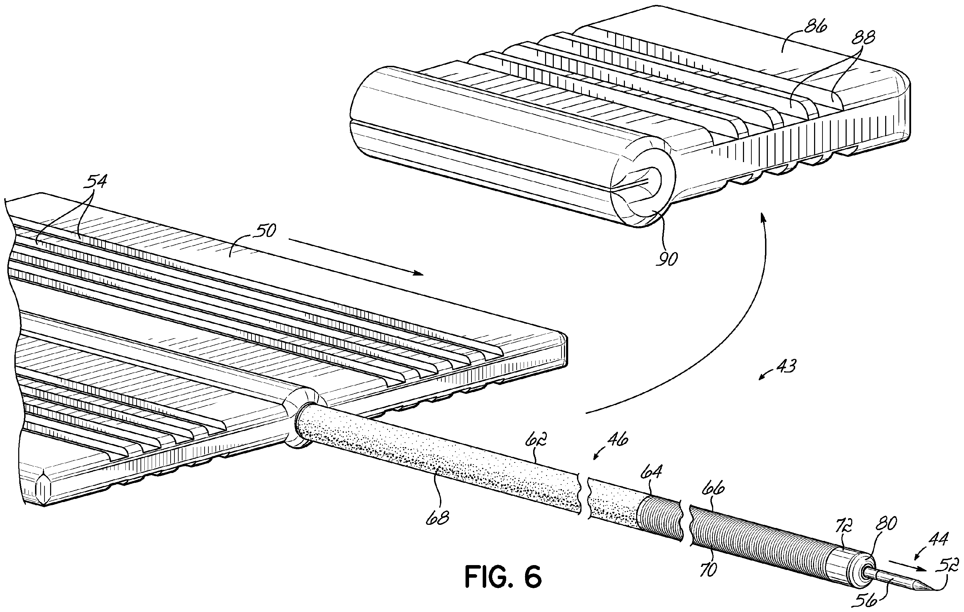

FIG. 6 illustrates the removal of the safety clip 86, which then allows the hub 50 of the piercing device 44 to advance distally and contact the proximal end of the coaxial guide-wire 46. As the hub 50 is advanced, the needle portion 52 extends distally from the radial tip 80 of the distal tip 72. Though not drawn to scale, from FIG. 6 is can be seen that the piercing device 44 can only extend distally from the radial tip 80 by an amount that is equal to the length of the attachment portion 90 that contacted the shaft 48 in FIG. 5.

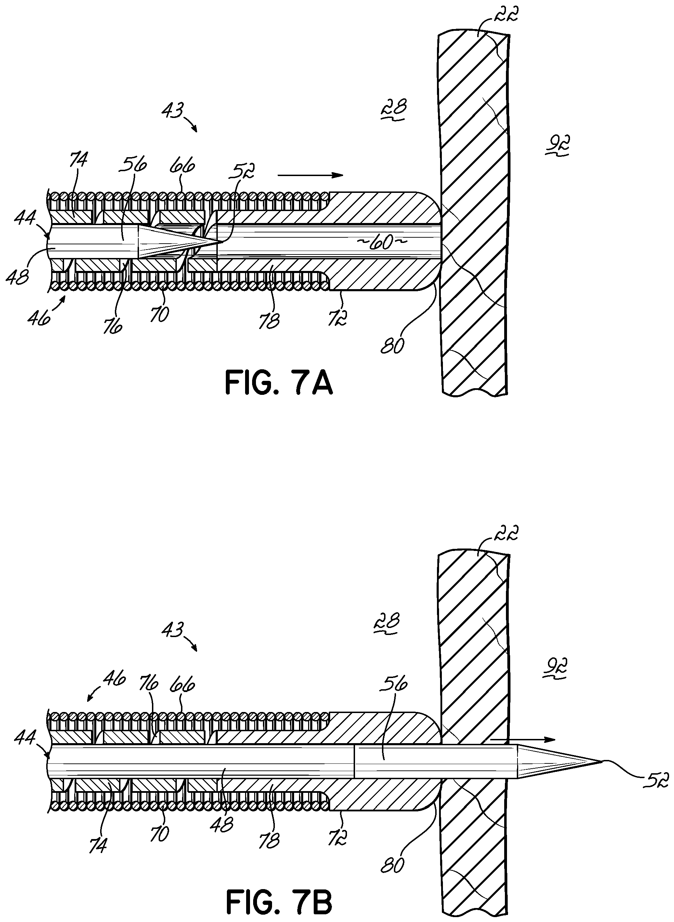

With the details of the coaxial transseptal device 43 described with some detail, one method of transseptal crossing can be described with reference to FIGS. 7A-7D.

FIG. 7A illustrates the coaxial transseptal device 43 as it is advanced into the right atrium 28 and such that the radial tip 80 contacts the intra-atrial septum 22. The needle portion 52 remains sheathed within the coaxial guide-wire 46.

In FIG. 7B, the physician can remove the safety clip 86 (FIGS. 5 and 6), if used, to release the shaft 48 such that it is distally moveable with respect to the coaxial guide-wire 46. The physician can then advance the piercing device 44, as had been shown previously in FIGS. 5 and 6, such that the hub 50 is advanced toward the proximal end of the coaxial guide-wire 46. Coincidentally, the needle portion 52 extends beyond the distal end of the radial tip 80 and punctures the intra-atrial septum 22. Continued advancement of the piercing device 44 causes the needle portion 52 to pass across the intra-atrial septum 22 and into the volume of the left atrium 92. During the piercing and crossing of the intra-atrial septum 22, the physician can constantly monitor the pressure within the left atrium with a pressure monitor via the removable adapter 94 (FIG. 2C).

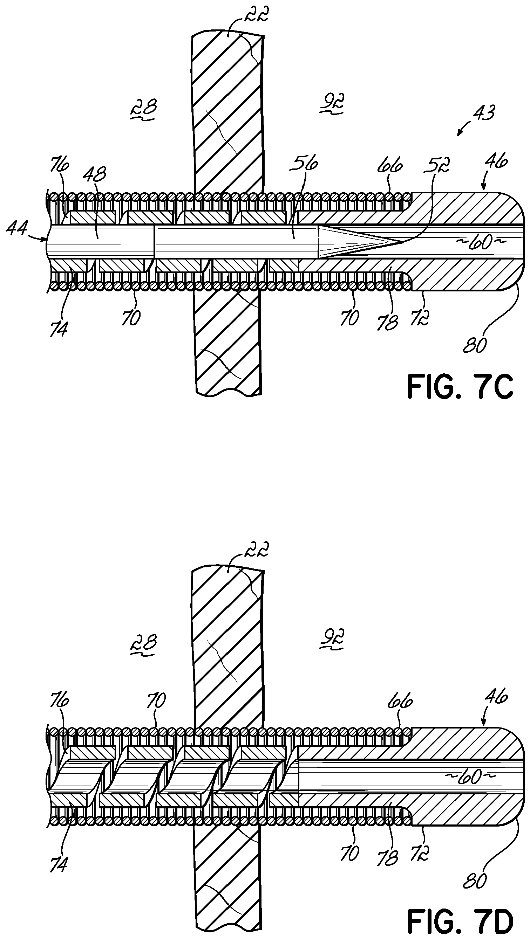

In FIG. 7C, the physician advances the coaxial guide-wire 46 across the intra-atrial septum 22 while maintaining the relative position of the piercing device 44 to the coaxial guide-wire 46 so as to not puncture additional tissues. The radial tip 80 can be used to dilate the puncture created by the piercing device 44 through the intra-atrial septum 22 to a diameter that is similar to the outer diameter of the coaxial guide-wire 46.

With the distal tip 72 of the coaxial guide-wire 46 in the left atrium 92, the physician can then retract the piercing device 44 from the coaxial guide-wire 46. The removable adapter 94 (FIG. 2C) is removed leaving only the coaxial guide-wire 46 in place and as shown in FIG. 7D. The coaxial guide-wire 46 is then prepared to receive auxiliary devices.

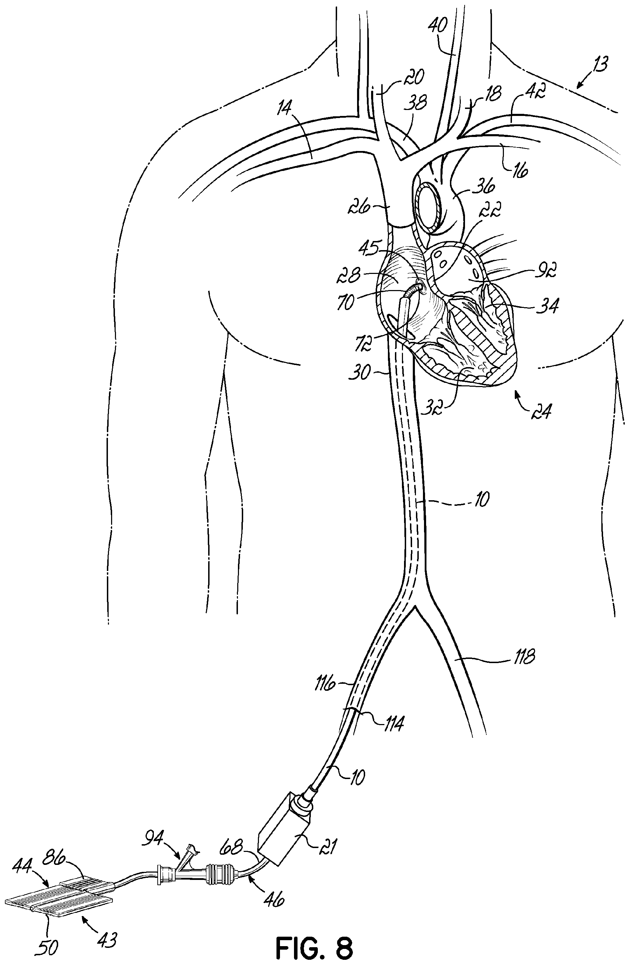

FIG. 8 illustrates an exemplary alternate method of accessing a tissue within the heart 24 of a patient 13. As shown, a vascular access site 114 can be chosen to be from an inferior location, such as the right or left femoral veins 116, 118. The guide catheter 10 and coaxial transseptal device 43 are then directed to the right atrium 28 from the inferior vena cava 30. The physician can then cross the intra-atrial septum 22 in a manner similar to the procedure described in detail above.

While the present invention has been illustrated by a description of various preferred embodiments and while these embodiments have been described in some detail, additional advantages and modifications will readily appear to those skilled in the art. The various features of the invention may be used alone or in any combination depending on the needs and preferences of the user.

## Claims

1. A coaxial transseptal device comprising a piercing device (44) comprising a shaft (48) having a proximal end, a distal end, and a sharpened portion (52) on the distal end, anda coaxial member (46) having a proximal end, a distal end, and a lumen extending between the proximal and distal ends of the member (46), the lumen of the member (46) configured to receive the piercing device (44) and to move relative thereto; **characterized in that** the coaxial member is a guide-wire having (46) a flexibility that increases distally, and **in that** a flexibility of the shaft (48) of the piercing device (44) increases distally, the shaft (48) of the piercing device (44) including a helical cut section (82) on a distal end thereof for increasing the flexibility.

2. The coaxial transseptal device of claim 1, wherein the guide-wire includes a tube (74) having a helical cut on a distal end thereof for increasing the flexibility.

3. The coaxial transseptal device of claim 2, wherein the helical cut has a pitch that varies along the tube (74), whereby the pitch is smaller at a distal end than at a proximal end of the helical cut.

4. The coaxial transseptal device of claim 2, wherein the guide-wire (46) includes a coil (70) surrounding at least the tube (74) of the guide-wire (46).

5. The coaxial transseptal device of claim 4, the helical cut of the tube (74) of the guide-wire (46) having a direction that opposes a direction of the coil (70)..

6. The coaxial transseptal device of claim 2, further comprising a sleeve (68) surrounding the proximal end of the tube (74) and configured to decrease the flexibility of the proximal end of the tube (74) relative to the distal end of the tube (74).

7. The coaxial transseptal device of claim 1, wherein the helical cut section of the shaft (48) of the piercing device (44) has a pitch that varies along the shaft (48), the pitch being smaller at a distal end than at a proximal end of the helical cut section.

8. The coaxial transseptal device of claim 1 wherein the helical cut section of the shaft (48) of the piercing device (44) has a depth that varies along the shaft (48), the depth being deeper at a distal end than at a proximal end of the helical cut section.

9. The coaxial transseptal device of claim 1, wherein the piercing device (44) includes a hub (50) on the proximal end of the shaft (48) that is adapted to enable the manipulation of the piercing device (44).

10. The coaxial transseptal device of claim 9 further comprisinga safety clip (86) configured to attach to the shaft (48) of the piercing device (44) between the hub (50) of the piercing device (44) and the proximal end of the guide-wire (46), wherein the safety clip (86) prevents the sharpened portion (52) of the piercing device (44) from extending beyond the distal end of the guide-wire (46).

11. The coaxial transseptal device of claim 1, further comprising an outer surface extending between the proximal and distal ends of the guide-wire (46) and configured to receive a tubular surgical device thereon such that the tubular surgical device slides relative thereto.

12. The coaxial transseptal device of claim 1, wherein the coaxial guide-wire includes a radial tip (80) at the distal end.

13. The coaxial transseptal device of claim 12, wherein the radial tip (80) is constructed from a radiopaque material.

14. The coaxial transseptal device of claim 1, wherein the coaxial guide-wire further comprises a removable adapter (94) connected to the proximal end of the coaxial guide-wire (46) and that couples to a pressure monitor for measuring a pressure within a chamber of the heart.

## Patentansprüche

1. Koaxiale transseptale Vorrichtung, umfassend eine Einstechvorrichtung (44), die einen Schaft (48) mit einem proximalen Ende, einem distalen Ende und einem geschärften Teil (52) auf dem distalen Ende umfasst, und ein koaxiales Element (46) mit einem proximalen Ende, einem distalen Ende und einem Lumen, das sich zwischen dem proximalen und distalen Ende des Elements (46) erstreckt, wobei das Lumen des Elements (46) konfiguriert ist, um die Einstechvorrichtung (44) aufzunehmen und sich relativ dazu zu bewegen, **dadurch gekennzeichnet, dass** das koaxiale Element ein Führungsdraht mit (46) einer Flexibilität ist, die distal zunimmt, und dass eine Flexibilität des Schafts (48) der Einstechvorrichtung (44) distal zunimmt, wobei der Schaft (48) der Einstechvorrichtung (44) einen spiralförmigen Schnittabschnitt (82) auf einem distalen Ende davon zum Erhöhen der Flexibilität aufweist.

2. Koaxiale transseptale Vorrichtung nach Anspruch 1, wobei der Führungsdraht eine Röhre (74) mit einem spiralförmigen Schnitt auf einem distalen Ende davon zum Erhöhen der Flexibilität aufweist.

3. Koaxiale transseptale Vorrichtung nach Anspruch 2, wobei der spiralförmige Schnitt eine Steigung hat, die entlang der Röhre (74) variiert, wodurch die Steigung an einem distalen Ende kleiner ist als an einem proximalen Ende des spiralförmigen Schnitts.

4. Koaxiale transseptale Vorrichtung nach Anspruch 2, wobei der Führungsdraht (46) eine Spule (70) aufweist, die mindestens die Röhre (74) des Führungsdrahts (46) umgibt.

5. Koaxiale transseptale Vorrichtung nach Anspruch 4, wobei der spiralförmige Schnitt der Röhre (74) des Führungsdrahts (46) eine Richtung hat, die einer Richtung der Spule (70) entgegengesetzt ist.

6. Koaxiale transseptale Vorrichtung nach Anspruch 2, die ferner eine Hülse (68) umfasst, die das proximale Ende der Röhre (74) umgibt und konfiguriert ist, um die Flexibilität des proximalen Endes der Röhre (74) relativ zu dem distalen Ende der Röhre (74) zu verringern.

7. Koaxiale transseptale Vorrichtung nach Anspruch 1, wobei der spiralförmige Schnittabschnitt des Schafts (48) der Einstechvorrichtung (44) eine Steigung hat, die entlang des Schafts (48) variiert, wobei die Steigung an einem distalen Ende kleiner ist als an einem proximalen Ende des spiralförmigen Schnittabschnitts.

8. Koaxiale transseptale Vorrichtung nach Anspruch 1, wobei der spiralförmige Schnittabschnitt des Schafts (48) der Einstechvorrichtung (44) eine Tiefe hat, die entlang des Schafts (48) variiert, wobei die Tiefe an einem distalen Ende tiefer ist als an einem proximalen Ende des spiralförmigen Schnittabschnitts.

9. Koaxiale transseptale Vorrichtung nach Anspruch 1, wobei die Einstechvorrichtung (44) eine Nabe (50) auf dem proximalen Ende des Schafts (48) aufweist, die ausgelegt ist, um die Handhabung der Einstechvorrichtung (44) zu ermöglichen.

10. Koaxiale transseptale Vorrichtung nach Anspruch 9, die ferner eine Sicherheitsklemme (86) umfasst, die konfiguriert ist, um an dem Schaft (48) der Einstechvorrichtung (44) zwischen der Nabe (50) der Einstechvorrichtung (44) und dem proximalen Ende des Führungsdrahts (46) angebracht zu sein, wobei die Sicherheitsklemme (86) verhindert, dass sich der geschärfte Teil (52) der Einstechvorrichtung (44) über das distale Ende des Führungsdrahts (46) hinaus erstreckt.

11. Koaxiale transseptale Vorrichtung nach Anspruch 1, die ferner eine äußere Fläche umfasst, die sich zwischen dem proximalen und distalen Ende des Führungsdrahts (46) erstreckt und konfiguriert ist, um eine rohrförmige chirurgische Vorrichtung darauf derart aufzunehmen, dass sich die rohrförmige chirurgische Vorrichtung relativ dazu verschiebt.

12. Koaxiale transseptale Vorrichtung nach Anspruch 1, wobei der koaxiale Führungsdraht eine radiale Spitze (80) an dem distalen Ende aufweist.

13. Koaxiale transseptale Vorrichtung nach Anspruch 12, wobei die radiale Spitze (80) aus einem strahlenundurchlässigen Material hergestellt ist.

14. Koaxiale transseptale Vorrichtung nach Anspruch 1, wobei der koaxiale Führungsdraht einen entfernbaren Adapter (94) umfasst, der mit dem proximalen Ende des koaxialen Führungsdrahts (46) verbunden ist und mit einem Druckwächter zum Messen eines Drucks innerhalb einer Kammer des Herzens gekoppelt ist.

## Revendications

1. Dispositif transseptal coaxial comprenant un dispositif de perçage (44) comprenant une tige (48) dotée d'une extrémité proximale, d'une extrémité distale, et d'une section aiguisée (52) sur l'extrémité distale, et un élément coaxial (46) doté d'une extrémité proximale, d'une extrémité distale, et d'un lumen s'étendant entre les extrémités proximale et distale de l'élément (46), le lumen de l'élément (46) étant conçu pour recevoir le dispositif de perçage (44) et pour bouger par rapport à celui-ci ; **caractérisé en ce que** l'élément coaxial est un guide-fil ayant (46) une flexibilité qui augmente au niveau distal, et **en ce qu'**une flexibilité de la tige (48) du dispositif de perçage (44) augmente au niveau distal, la tige (48) du dispositif de perçage (44) comportant une section à découpe hélicoïdale (82) sur son extrémité distale pour augmenter la flexibilité.

2. Dispositif transseptal coaxial selon la revendication 1, dans lequel le guide-fil comporte un tube (74) doté d'une découpe hélicoïdale sur son extrémité distale pour augmenter la flexibilité.

3. Dispositif transseptal coaxial selon la revendication 2, dans lequel la découpe hélicoïdale a un pas qui varie le long du tube (74), le pas étant plus réduit à une extrémité distale qu'à une extrémité proximale de la découpe hélicoïdale.

4. Dispositif transseptal coaxial selon la revendication 2, dans lequel le guide-fil (46) comporte une boucle (70) entourant au moins le tube (74) du guide-fil (46).

5. Dispositif transseptal coaxial selon la revendication 4, la découpe hélicoïdale du tube (74) du guide-fil (46) ayant un sens opposé à un sens de la boucle (70).

6. Dispositif transseptal coaxial selon la revendication 2, comprenant un manchon (68) entourant l'extrémité proximale du tube (74) et conçu pour réduire la flexibilité de l'extrémité proximale du tube (74) par rapport à l'extrémité distale du tube (74).

7. Dispositif transseptal coaxial selon la revendication 1, dans lequel la section à découpe hélicoïdale de la tige (48) du dispositif de perçage (44) a un pas qui varie le long de la tige, le pas étant plus réduit à une extrémité distale qu'à une extrémité proximale de la section à découpe hélicoïdale.

8. Dispositif transseptal coaxial selon la revendication 1, dans lequel la section à découpe hélicoïdale de la tige (48) du dispositif de perçage (44) a une profondeur qui varie le long de la tige (48), la profondeur étant plus importante à une extrémité distale qu'à une extrémité proximale de la section à découpe hélicoïdale.

9. Dispositif transseptal coaxial selon la revendication 1, dans lequel le dispositif de perçage (44) comporte sur l'extrémité proximale de la tige (48) une plate-forme (50) qui est apte à permettre la manipulation du dispositif de perçage (44).

10. Dispositif transseptal coaxial selon la revendication 9, comprenant en outre un clip de sécurité (86) conçu pour être attaché à la tige (48) du dispositif de perçage (44) entre la plate-forme (50) du dispositif de perçage (44) et l'extrémité proximale du guide-fil (46), le clip de sécurité (86) empêchant la section aiguisée (52) du dispositif de perçage (44) de s'étendre au-delà de l'extrémité distale du guide-fil (46).

11. Dispositif transseptal coaxial selon la revendication 1, comprenant en outre une surface extérieure s'étendant entre les extrémités proximale et distale du guide-fil (46) et conçue pour recevoir à l'intérieur un dispositif chirurgical tubulaire de manière à ce que le dispositif chirurgical tubulaire glisse par rapport à elle.

12. Dispositif transseptal coaxial selon la revendication 1, dans lequel le guide-fil coaxial comprend une pointe radiale (80) à son extrémité distale.

13. Dispositif transseptal coaxial selon la revendication 12, dans lequel la pointe radiale (80) est réalisée dans un matériau radio-opaque.

14. Dispositif transseptal coaxial selon la revendication 1, dans lequel le guide-fil coaxial comprend en outre un adaptateur amovible (94) connecté à l'extrémité proximale du guide-fil coaxial (46) et qui se couple à un moniteur de pression pour mesurer une pression dans une chambre du coeur.
